# EUROPEAN PATENT SPECIFICATION

(11) **EP 1 514 517 B1**
(45) Date of publication and mention of the grant of the patent: **26.03.2008**
(21) Application number: 04021563.4
(22) Date of filing: 10.09.2004
(51) Int. Cl.: A61B 17/06

(54) **Method for treating a section of a suture and forming a suture tip for attachment to a needle**
Verfahren zur Behandlung von einem Abschnitt eines Nähfadens und zur Bildung einer Nähfadenspitze zum Befestigen an eine Nadel
Procédé de traitement d'une section de suture et de formation de l'extrémité d'une suture pour attacher à une aiguille

(30) Priority: 10.09.2003 US 501635 P
(43) Date of publication of application: 16.03.2005
(73) Proprietor: Tyco Healthcare Group LP, Norwalk, Connecticut 06856 (US)
(72) Inventor: Roby, Mark S., Killingworth CT 06419 (US); Maiorino, Nicholas, Branford CT 06405 (US)
(74) Representative: HOFFMANN EITLE

(56) References cited:
- US-A- 3 980 177
- US-A- 5 250 247
- US-A- 5 259 845
- US-A- 5 269 808

## Description

### BACKGROUND

### Technical Field

The present disclosure generally relates to tipping surgical sutures. More particularly, the present disclosure is directed to a method for removing the coating from a section of suture and then applying a tipping agent to the uncoated section of suture. For disclosures of methods of tipping surgical sutures, reference may be made to US-A-3980177 and US-A-5269808.

### Background of Related Art

For many years, surgeons have employed needle-suture combinations in which a suture or ligature is attached to the shank end of a needle. Such needle-suture combinations are provided for a wide variety of monofilament and braided suture materials, both absorbable and non-absorbable, e.g., catgut, silk, nylon, polyester, polypropylene, linen, cotton, and absorbable synthetic materials such as polymers and copolymers of glycolic and lactic acid.

Needle-suture combinations fall into two general classes: standard, or non-detachable, needle attachment and removable, or detachable, needle attachment. In the case of standard needle attachment, the suture is securely attached to the needle and is not intended to be separable therefrom, except by cutting or severing the suture. Removable needle attachment, by contrast, is such that the needle is separable from the suture in response to a force exerted by the surgeon. Minimum acceptable forces required to separate a needle from a suture (for various suture sizes) are set forth in the U.S. Pharmacopoeia ("USP"). As to detachable needles, the USP prescribes individual pull-out forces and average pull-out forces as measured for five needle-suture combinations. The pull-out forces for both standard and removable needle-suture attachments set forth in the USP are incorporated by reference herein.

One typical method for securing a suture to a needle involves providing a cylindrical recess in the shank end of a needle and securing a suture therein. For example, U.S. Pat. No. 1,558,037 teaches the addition of a cement material to a substantially cylindrical recess to secure the suture therein. Additional methods for bonding a suture within a needle bore are described in U.S. Pat. Nos. 2,928,395 (adhesives) and 3,394,704 (bonding agents). Alternatively, a suture may be secured within an axial bore in a needle by swaging the needle in the region of the recess. See, e.g., U.S. Pat. No. 1,250,114. Additional prior art methods for securing a suture within a needle bore include expansion of a catgut suture through the application of heat (U.S. Pat. No. 1,665,216), inclusion of protruding teeth within the axial bore to grasp an inserted suture (U.S. Pat No. 1,878,361) and knotting the end of the suture to be inserted within the bore to secure the suture therein (U.S. Pat No. 1,757,129).

Insertion of sutures into a hole, recess or tube for attachment to surgical needles presents problems peculiar to needle-suture combinations. Braided multifilament sutures in particular are difficult to insert into the very small aperture of a surgical needle: unless modified, they are too limp for the suture tip to be controlled for insertion and they have a tendency to "broom", i.e., the filaments have a tendency to flare out at the cut end so that the diameter of the cut end exceeds the diameter of the needle hole. Various techniques have been employed to modify sutures to overcome the problems of limpness and brooming. One known method employs a tipping agent, which is a material used to coat the suture to stiffen the filaments and adhere them together so that the suture may be more easily inserted into the shank end of a surgical needle.

Typically, a suture to be tipped is first placed under tension to reduce slack so that the suture may be maintained in a predetermined position on a frame, drum, rack or other suture holding device. Optionally, the tension may be such as to reduce the diameter of the suture. See, Canadian Patent No. 1,009,532. The suture is then dipped into the tipping solution and allowed to dry while under tension. The sutures are dried, for example, by being warmed in a drying oven at about 107°C (225°F) for about 10 minutes. After drying the sutures can be cut and released from tension. The process results in a tipped section on each side of the cut, with the tipping agent adhering the suture filaments to one another to prevent brooming. The tipped section facilitates insertion of the suture end into a needle bore. Where tension has optionally been employed to reduce the suture diameter, release of said tension will allow the suture to expand to its original diameter except at the tipped section. This can further facilitate insertion of the cut end of the suture into a needle.

Tipping agents may be dissolved in solvents to form dipping solutions. By way of example, Mariotte mixture is a dipping solution comprising nylon dissolved in isopropyl alcohol. Other polymers and solvents may also be used. Gould mixture is a dipping solution comprising nylon dissolved in methanol. At least one major manufacturer of surgical needles recommends use of Mariotte mixture or Gould mixture for tipping sutures. A multitude of other tipping agents, including polymers and solvents, have been proposed. For example, McGregor U.S. Pat. No. 3,890,975 discloses coating the suture with a binding resin or adhesive.

Schmitt U.S. Pat. No. 3,736,646 discloses that it is known to tip braided sutures by dipping the end of the suture in plastic such as a polymer solution in isopropyl alcohol. Schmitt suggests that for absorbable sutures an absorbable tipping agent is desirable, and proposes that a copolymer of lactic and glycolic acid dissolved in a suitable organic solvent, such as xylene or toluene, be applied to tip the suture.

Nichols U.S. Pat. No. 2,734,506 discloses a dipping solution of polymers of methacrylic acid esters in an organic solvent such as toluene, xylene, acetone, ethyl acetate, methyl ethyl ketone, or naphtha.

Shepherd et al. U.S. Pat. No. 3,849,185 discloses the use of an acrylic casting syrup as a tipping agent, the syrup being fully polymerized after application to the suture.

An improved method for tipping sutures is disclosed and described in above-mentioned U.S. Pat. No. 5,269,808 to Proto et al. This method includes winding the suture around a drum while continuously monitoring the suture diameter and adjusting the tension on the suture to control the suture diameter as it is being wound. The drum is then placed into an apparatus which passes the section of the suture to be tipped through a mist of cyanoacrylate tipping agent generated by an ultrasonically powered nozzle. The cyanoacrylate tipping agent hardens very rapidly after being absorbed into the braided suture and adheres the filaments so that the filaments will not broom when the tipped section is cut for insertion into a surgical needle to form a needle-suture device. One of the advantages of this method is the cyanoacrylate works well on sutures which have previously been coated and/or filled with lubricants, and/or therapeutic agents and the like.

U.S. Pat. No. 5,156,788 to Chesterfield et al., discloses a method and apparatus for tipping sutures by delimiting a section of the suture and heating the delimited section under predetermined time and temperature conditions to reversibly stiffen the delimited section upon cooling. The cooled, stiffened suture section is cut to provide a suitably stiffened, non-brooming suture tip for insertion into a needle.

Although tipped sutures prepared in accordance with the above procedures have been used successfully, there are several drawbacks with the use of tipping solutions. The main problems relate to tipping consistency and process control. Non-uniform solvent evaporation, which may be caused by variations in the solvent, oven temperature and heating time, can result in inconsistent tipping. Furthermore, the dried residue of polymer left after evaporation can flake off or develop cracks. Additionally, an excess length of suture must be tipped to provide a sufficient length of stiff suture for handling during insertion into the needle. However, this undesirably results in a stiff suture segment adjacent the needle.

A further consideration pertinent to suture tipping is that sutures are often prepared with lubricant coatings such as absorbable polymers, silicone or fatty acid salts in order to increase lubricity and to improve "tie-down" performance, i.e., the ease of sliding a knot down the suture into place. Such lubricant coatings may be incompatible with the materials and methods currently employed for tipping sutures. In particular, most prior known tipping agents, other than cyanoacrylate, do not adhere well to lubricant coated sutures, which may result in inconsistent tipping or an undesirable reduction of needle-suture pull out force.

Where tipping agents are to be applied to a coated suture, it would be desirable to remove the coating from the area of the suture to be tipped or utilize a tipping agent capable of coating over existing suture coatings to reduce processing steps and simplify manufacture. Prior art methods, such as those disclosed in U.S. Pat. Nos. 5,269,808 and 5,259,845, utilize tipping agents capable of coating over existing suture coats.

US-A-3980177 discloses a method for tipping a suture comprising: providing a suture with a wax coating; removing at least a portion of wax coating to provide an uncoated portion of the suture; and applying a tipping agent to the uncoated portion of the suture.

### SUMMARY

In accordance with the present disclosure, there is provided a method for removing the coating from the section of a suture to be tipped to provide improved adherence of the suture to the needle. Sutures treated in accordance with the present disclosure are suitable for use in a combined surgical needle-suture device.

The invention is defined in claim 1 and the dependent claims are directed to optional and preferred features of the invention.

### BRIEF DESCRIPTION OF THE DRAWINGS

FIG. 1 is a perspective view of a tipping drum, 26. possessing an axial spindle 29 upon which it can turn, a smooth external surface 31, and channel 32 extending lengthwise along the drum.
FIG. 2 depicts a wound tipping drum 26 viewed from its end which shows suture material 30 wound around the external surface of the drum 31. A metal bar 27 that has been wrapped with coating absorbent paper 28 has been inserted into the drum's channel 32.
FIG. 3 is a side view of the wound tipping drum 26 with the metal bar 27 wrapped 25 with coating absorbent paper 28 that has been inserted through the channel 32 of the tipping drum. The ends of the metal bar are placed on supports 33 and 34, and a second piece of coating absorbent paper 35 has been placed over the external surface of the suture 30 lying directly over the channel 32.

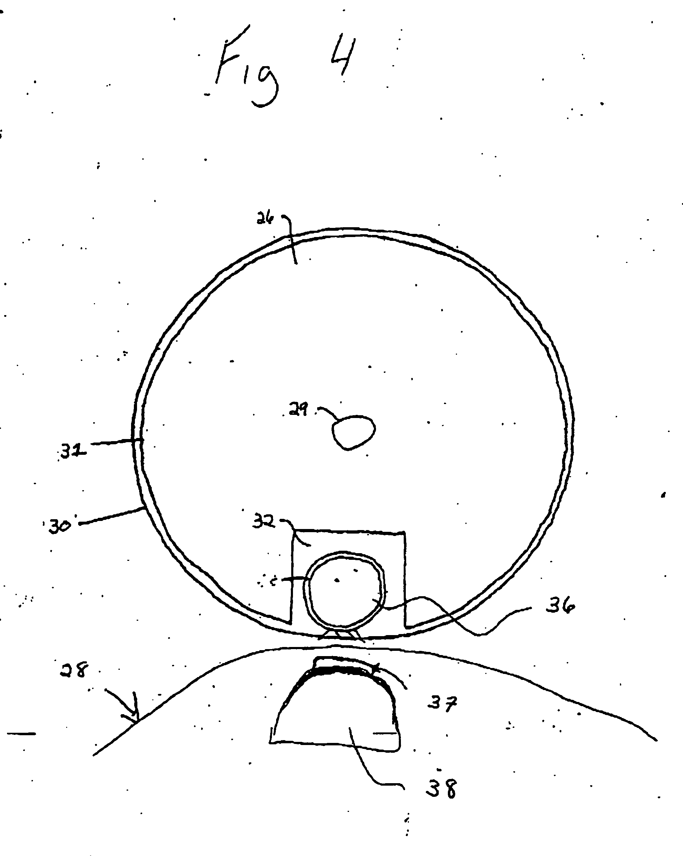

FIG. 4 depicts a wound tipping drum 26 viewed from its end which shows suture material 30 wound around the external surface of the drum. A hot air manifold 36 has been inserted into the drum's channel 32. Underneath the tipping drum is a piece of coating absorbent paper 28 overlying a foam material 37 which, in turn, lies over a metal bar 38.

### DESCRIPTION OF THE PREFERRED EMBODIMENTS

The present disclosure is directed to a method for tipping sutures, especially coated multifilament braided sutures. As used herein, the term "braided" means a strand formed by crossing a number (at least three) of individual strands composed of one or more filaments diagonally in such a manner that each strand passes alternatively over and under one or more of the others. The braid may be of traditional tubular braid construction or spiroid braid construction and may include a core section composed of one or more filaments around which the braid is externally fabricated. Suitable braid constructions are disclosed, for example, in U.S. Pat. Nos. 5,261,886; 5,059,213; 5,019,093; 3,565,077; and 3,187,752.

The suture can be fabricated from a wide variety of natural and synthetic fibrous materials. Such materials include non-absorbable as well as partially and fully bio-absorbable (i.e., resorbable) natural and synthetic fiber-forming polymers. Non-absorbable materials which are suitable for fabricating sutures include polyamides, polyesters and natural materials Including silk. Bio-absorbable sutures may be fabricated from natural collagenous material or synthetic resins including those derived from glycolic acid, glycolide, lactic acid, lactide, dioxanone, polycaprolactone, epsilon-caprolactone, trimethylene carbonate, etc., and various combinations of these and related monomers. Sutures prepared from resins of this type are known in the art. See, e.g., U.S. Pat. Nos. 3,297,033; 3,839,297; and 4,429,080.

Braided multifilament sutures typically are coated with one or more coating compositions to improve functional properties such as surface lubricity and knot tie-down behavior. A variety of suture coating compositions proposed for either or both of these purposes are well known in the art, e.g., those disclosed in U.S. Pat. Nos. 3,867,190; 3,942,532; 4,047,533; 4,452,973; 4,624,256; 4,649,920; 4,716,203; 4,826,945,4,994,074; 5,123,912, and 5,312,437.

Absorbable braided sutures may also contain a storage stabilizing amount of a filler material containing at least one Water soluble liquid polyhydroxy compound and/or ester thereof. In addition to having an enhanced degree of storage stability, a braided suture which has been filled with a storage stabilizing amount of, e.g., glycerol, exhibits better flexibility and "hand" characteristics than the untreated suture. Suitable filling compositions are disclosed in U.S. Pat Nos. 5,051,272 and 5,037,429.

A braided suture may also be impregnated with one or more medico-surgically useful substances, e.g., those which accelerate or beneficially modify the healing process when the suture is applied to a wound or surgical site. So, the braided suture herein can be provided with a therapeutic agent which will be deposited at the sutured site. For example, a therapeutic agent can be chosen for its antimicrobial properties, capability for promoting wound repair and/or tissue growth, or for specific indications such as thrombosis. Antimicrobial agents such as broad spectrum antibiotics (gentamicin sulphate, erythromycin or derivatized glycopeptides) which are slowly released into the tissue can be applied in this manner to aid in combating clinical and sub-dinical infections in a surgical or trauma wound site. To promote wound repair and/or tissue growth, one or more biologically active materials known to achieve either or both of these objectives can be applied to the braided suture of the present invention. Such materials include any of several Human Growth Factors (HGFs), magainin, tissue or kidney plasminogen activator to cause thrombosis, superoxide dismutase to scavenge tissue-damaging free radicals, tumor necrosis factor for cancer therapy, colony stimulating factor, interferon, interleukin-2 or other lymphokine to enhance the immune system, and so forth.

In a preferred embodiment, sutures to be treated in accordance with the present disclosure are silk sutures coated with wax. In accordance with the present disclosure, the suture is first wound onto a suture holding device, such as a frame, drum, or rack, commonly used in the tipping process. Heat is then applied to that section of the suture to which tipping agent will be applied at a sufficient temperature and for a sufficient time to at least partially melt the coating, but insufficient to structurally degrade the suture material. Heat may be applied by heating means known to those skilled in the art including, but not limited to, an iron, a heat gun, a hot air manifold, etc. The melted coating is then collected utilizing a porous substrate, such as a sponge, a coating absorbent paper, or fabric. The suture is then ready to undergo a tipping process whereby a tipping agent is applied to the uncoated section of the suture. The tipped section of suture may then be cut and the tipped end attached to the shank end of a surgical needle to form a needle-suture combination.

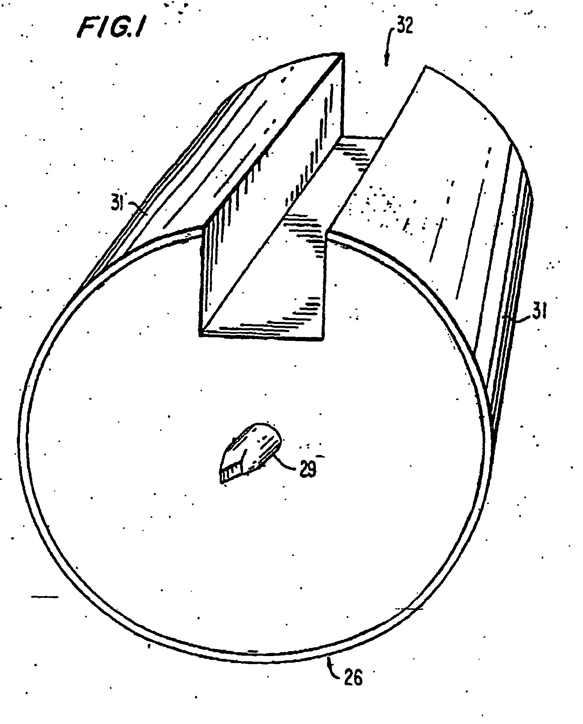

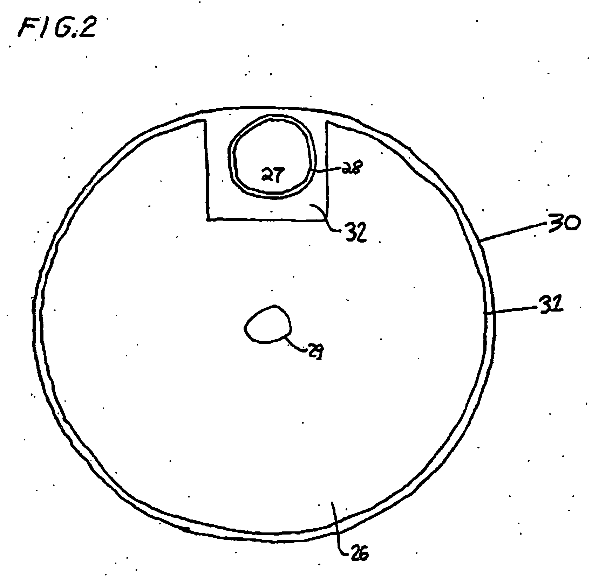

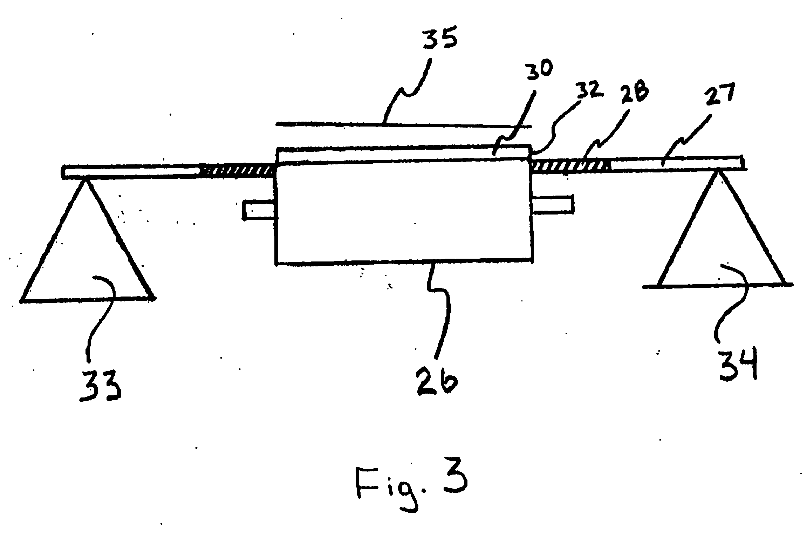

In one embodiment, a tipping drum is used to hold the suture to be tipped. Referring to FIGS. 1-3, in a preferred embodiment, the drum 26 possesses a channel 32 on its outer surface 31 that corresponds to the section of suture that will be tipped. Accordingly, the suture 30 on a wound tipping drum possesses both an external surface and an internal surface: while most of the internal surface of the suture is in contact with the surface of the drum, the internal surface of the suture lying over the channel is not in contact with any portion of the drum and is exposed to, and accessible from, the channel of the drum.

After the drum has been wound with coated suture, a metal bar 27, preferably made of aluminum, is wrapped with a coating absorbent paper 28 and inserted into the channel 32 in the drum 26. The ends of the metal bar are then placed on supports 33 and 34 so that the paper-wrapped metal bar supports the weight of the wound tipping drum and is in contact with the internal surface of the suture lying over the channel.

In one embodiment, a second piece of coating absorbent paper 35 is then placed over the outer surface of the suture covering the channel, and a heat source, e.g. an iron, is placed onto the second piece of coating absorbent paper for sufficient time to at least partially melt the coating from the section of the suture lying over the channel, but insufficient to structurally degrade the suture material. It is preferred to heat the iron to a temperature ranging from about 100° C to about 265°C. Most preferably, the temperature range for use with silk sutures is 100° C to 200°C, which has been unexpectedly found to ensure maximum pull strength. The iron is placed onto the second piece of coating absorbent paper for a time ranging from about 1 second to about 60 seconds to melt the coating. The coating absorbent paper on the metal bar in the channel and the second piece of coating absorbent paper on the external surface of the suture collect the melted coating. Once the metal bar wrapped with coating absorbent paper is removed from the channel and the second piece of coating absorbent paper is removed from the external suture surface, tipping agent may then be applied to the now uncoated section of suture.

In another embodiment, a heat gun is used as a heat source instead of an iron. Where a heat gun is used, the second piece of coating absorbent paper discussed above is not required. The air from the heat gun is heated to a temperature ranging from about 100°C to about 265°C, and more preferably to a temperature range from about 100° C to about 200° C. The heat is then directed onto the external surface of the suture. The heat gun is placed at a distance ranging from about 2.5 mm (0.1 inches) to about 6.25 cm (2.5 inches) from the section of the suture lying over the channel. In a preferred embodiment, the suture is subjected to the heated air for a time period ranging from about 5 seconds to about 3 minutes. The coating on the external surface of the suture either falls off or migrates to the internal surface of the suture. The metal bar wrapped with coating absorbent paper wicks the coating material from the internal surface of the suture.

In yet another embodiment, a hot air manifold 36 is inserted into the channel 32 of the wound tipping drum and heated air is directed onto the internal surface of the suture lying over the channel. In this embodiment, the drum is placed upside down so that the channel is at the bottom of the tipping drum. The hot air manifold is inserted into the channel and air blows down onto the internal surface of the suture. The wound tipping drum is placed onto a piece of paper 28, which in turn lies over a metal bar 38. Gravitational forces assist the paper in wicking the wax from the internal and external suture surfaces. In a preferred embodiment, a foam material 37, such as Armorflex™ is placed between the paper and metal bar. It is preferred that the air be heated to a temperature ranging from about 100°C to about 265°C, and more preferably to a range between about 100°C and 200°C. The hot air manifold is placed at a distance from the suture ranging from about 2.5 mm (0.1 inches) to about 5 cm (2 inches). In a preferred embodiment, the suture is subjected to the heated air for a time period ranging from about 5 seconds to about 3 minutes.

Once the coating has been removed from the section of the suture lying over the channel, the tipping agent is applied to the uncoated section in accordance with standard techniques known to those skilled in the art. Once the tipping process is complete, the tipped section is ready to be cut and attached to a surgical needle. The resulting needle-suture combination demonstrates improved adherence of the tipping agent to the suture to facilitate attachment of the cut end to the shank of a needle and improved pull-out forces.

The following non-limiting examples are illustrative of the sutures and the method for their manufacture in accordance with the present disclosure.

### EXAMPLE 1

A 2/0 wax-coated silk suture was wound onto a tipping drum with a 5 cm x 5 cm (2" by 2") channel that permitted the application of tipping agent. An aluminum bar wrapped with paper was then inserted into the channel and the ends of the aluminum bar were placed on supports so that the aluminum bar was in contact with the inner surface of the suture lying over the channel, and the aluminum bar supported the weight of the tipping drum. A second piece of paper was placed over the outer surface of the section of suture covering the channel. An iron that had been heated to 250°C was then applied to the second piece of paper for 5 seconds. The second piece of paper was then removed from the suture material and the aluminum bar wrapped with paper was removed from the channel of the tipping drum. Both the paper on the aluminum bar and the second piece of paper had wax on them, demonstrating the removal of the coating from the surface of the suture. The drum was then placed into a tipping machine and tipping agent was applied to the uncoated section of the suture with excellent results.

### EXAMPLE 2

This example followed the same general process as Example 1 with minor modifications. In this example, the aluminum bar was wrapped with Armorflex™ foam, which was then wrapped with paper and inserted into the tipping drum's channel. A second piece of paper was not applied to the outer surface of the suture. A heat gun was used instead of an iron to melt the suture coating. The heat gun was placed at a distance of 25.4 mm (1 I inch) from the external surface of the suture material for times ranging from 30 seconds to 1 minute at temperatures of 265°C. Table 1 below shows the pull-out forces for sutures treated with a heat gun at 265°C, at a distance of 25.4 mm (1 inch).

**TABLE 1**

| 2/0 suture treated with a heat gut at 265°C at a distance of 25.4 mm (1 inch) | |
|---|---|
| | Pull-Out Force |
| 2 Passes | 2.59 |
| | 3.404 |
| | 2.762 |
| Trimmed Top | 2.390 |
| | 2.868 |
| | 3.018 |
| | 3.040 |
| | 3.268 |
| Trimmed | 2.230 |
| | 2.410 |
| | 2.432 |
| | 1.562 |
| | 1.702 |

### EXAMPLE 3

This example followed a procedure similar to Examples 1 and 2 except that in this example, a hot air manifold was used to heat the wax coating and gravity assisted in the removal of the wax. In this example, the wound tipping drum was oriented so that the channel was along the bottom of the drum. The hot air manifold was inserted into the channel of the drum and the drum was then placed onto a piece of paper lying over a piece of Armorflex™ foam which, in turn, was lying over a metal bar. The air was heated to 175°C and was applied to the internal surface of the drum for a period of 1 minute. Gravitational forces wicked the wax coating onto the paper so that the resulting suture had its coating removed from that section of the suture lying adjacent to the channel. Table 2 below shows the pull-out forces for sutures treated in accordance with this example.

**TABLE 2**

| Pull out Forces for Sutures |
|---|
| 3.250 |
| 3.172 |
| 3.030 |
| 2.276 |
| 2.086^{a} |
| 3.782^{a} |
| 2.970 |
| 2.634 |
| 2.540 |
| 2.470 ^{b} |
| 3.190^{b} |
| 2.328^{c} |
| 2286^{c} |
| 2.872 |
| 3.748 |
| 2.808 |
| 2.220^{d} |
| 3.112^{d} |
| 3.590 |

| |
|---|
| a = Same Suture, Trimmed Back b = Same Suture c = Same Suture d = Same Suture |

It will be understood that various modifications may be made to the embodiments disclosed herein. Therefore, the above description should not be construed as limiting, but merely as exemplifications of preferred embodiments. Those skilled in the art will envision other modifications within the scope of the claims appended hereto.

## Claims

1. A method for tipping a suture comprising:
providing a suture with a wax coating;
removing at least a portion of wax coating to provide an uncoated portion of the suture; and
applying a tipping agent to.the uncoated portion of the suture;
wherein the step of removing at least a portion of the wax coating comprises heating at least a portion of the coated suture to melt at least a portion of the wax coating and contacting the melted wax with an adsorbent or absorbent material.

2. The method of claim 1, further comprising the step of placing the suture on a suture holding device.

3. The method of claim 2, wherein the suture holding device is a drum.

4. The method of claim 1, 2 or 3, wherein the absorbent material is paper or fabric.

5. The method of any one of the preceding claims, wherein the step of heating comprises heating to a temperature in the range of from 100°C to 265°C.

6. The method of claim 5, wherein the step of heating comprises heating to a temperature in the range of from 100°C to 200°C.

7. The method of any one of the preceding claims, wherein the step of heating comprises applying heat for a period of time ranging from 1 second to 5 minutes.

8. The method of any one of the preceding claims, wherein the heating step comprising applying heat by directing heated air from a heat gun onto at least a portion of the suture.

9. The method of claim 8, wherein the heat gun is placed at a distance from 2.5 mm (0.1 inches) to 6.25 cm (2.5 inches) from the suture.

10. The method of any one of the preceding claims, wherein the step of providing a suture with a wax coating comprises providing as silk suture coated with wax

11. The method according to any one of the preceding claims, wherein the step of applying a tipping agent comprises applying a cyanoacrylate composition.

## Patentansprüche

1. Verfahren zum Ausbilden einer Spitze an einer chirurgischen Naht, umfassend:
Versehen einer chirurgischen Naht mit einer Wachsbeschichtung;
Entfernen mindestens eines Teils der Wachsbeschichtung, um einen nicht beschichteten Abschnitt der Naht bereitzustellen; und
Aufbringen eines Spitzen ausbildenden Mittels auf dem nicht beschichteten Teil der chirurgischen Naht;
wobei der Schritt des Entfernens von mindestens einem Teil der Wachsbeschichtung das Erwärmen mindestens eines Teils der beschichteten chirurgischen Naht umfasst, um mindestens einen Teil der Wachsbeschichtung zu schmelzen, und das In-Kontakt-Bringen des geschmolzenen Wachses mit einem adsorbierenden oder absorbierenden Material umfasst.

2. Verfahren nach Anspruch 1, weiter umfassend den Schritt des Platzierens der chirurgischen Naht auf eine Nahthaltevorrichtung.

3. Verfahren nach Anspruch 2, wobei die Nahthaltevorrichtung eine Trommel ist.

4. Verfahren nach Anspruch 1, 2 oder 3, wobei das absorbierende Material Papier oder Stoff ist.

5. Verfahren nach einem der vorangehenden Ansprüche, wobei der Schritt des Erwärmens das Erwärmen auf eine Temperatur im Bereich von 100°C bis 265°C umfasst.

6. Verfahren nach Anspruch 5, wobei der Schritt des Erwärmens das Erwärmen auf eine Temperatur im Bereich von 100°C bis 200°C umfasst.

7. Verfahren nach einem der vorangehenden Ansprüche, wobei der Schritt des Erwärmens das Anwenden von Wärme für einen Zeitraum im Bereich von einer Sekunde bis fünf Minuten umfasst.

8. Verfahren nach einem der vorangehenden Ansprüche, wobei der Erwärmungsschritt das Anwenden von Wärme durch Lenken von erwärmter Luft aus einer Heißluftpistole auf mindestens einen Teil der chirurgischen Naht umfasst.

9. Verfahren nach Anspruch 8, wobei die Heißluftpistole unter einem Abstand von 2,5 mm (0,1 Zoll) bis 6,25 cm (2,5 Zoll) von der chirurgischen Naht platziert wird.

10. Verfahren nach einem der vorangehenden Ansprüche, wobei der Schritt des Versehens einer chirurgischen Naht mit einer Wachsbeschichtung das Bereitstellen einer mit Wachs beschichteten Seidennaht umfasst.

11. Verfahren nach einem der vorangehenden Ansprüche, wobei der Schritt des Aufbringens eines Spitzen ausbildenden Mittels das Aufbringen einer Cyanoakrylat-Verbindung umfasst.

## Revendications

1. Procédé de formation de l'extrémité d'une suture comprenant:
appliquer à la suture un revêtement en cire;
retirer au moins une portion du revêtement de cire pour réaliser une portion non revêtue de la suture; et
appliquer un agent de formation d'extrémité à la portion non revêtue de la suture;
où l'étape consistant à retirer au moins une portion du revêtement de cire comprend l'échauffement d'au moins une portion de la suture revêtue pour faire fondre au moins une portion de revêtement de cire et pour mettre en contact la cire fondue avec un matériau adsorbant ou absorbant.

2. Procédé selon la revendication 1, comprenant en outre l'étape consistant à placer la suture sur un dispositif de retenue de suture.

3. Procédé selon la revendication 2, où le dispositif de retenue de suture est un tambour.

4. Procédé selon la revendication 1, 2 ou 3, où le matériau absorbant est du papier ou une étoffe.

5. Procédé selon l'une des revendications précédentes, où l'étape d'échauffement comprend l'échauffement à une température dans la plage de 100°C à 265°C.

6. Procédé selon la revendication 5, où l'étape d'échauffement comprend l'échauffement à une température dans la plage de 100°C à 200°C.

7. Procédé selon l'une des revendications précédentes, où l'étape d'échauffement comprend l'application de la chaleur pendant une période de temps s'étendant de 1 seconde à 5 minutes.

8. Procédé selon l'une des revendications précédentes, où l'étape d'échauffement comprend l'application de la chaleur en dirigeant de l'air chauffé par un pistolet thermique sur au moins une portion du matériau de suture.

9. Procédé selon la revendication 8, où le pistolet thermique est placé à une distance de 2,5 mm (0,1 pouce) à 6,25 cm (2,5 pouces) du matériau de suture.

10. Procédé selon l'une des revendications précédentes, où l'étape consistant à munir un matériau de suture d'un revêtement en cire comprend la réalisation d'un matériau de suture en soie revêtu de cire.

11. Procédé selon l'une des revendications précédentes, où l'étape consistant à appliquer un agent de formation d'extrémité comprend l'application d'une composition de cyanoacrylate.
